# EUROPEAN PATENT SPECIFICATION

(11) **EP 1 723 239 B1**
(45) Date of publication and mention of the grant of the patent: **09.12.2009**
(21) Application number: 05713436.3
(22) Date of filing: 09.02.2005
(51) Int. Cl.: C12N 15/62, C12N 15/85, C07K 14/705

(54) **METHOD FOR GENERATING TETHERED PROTEINS**
VERFAHREN ZUR ERZEUGUNG VERANKERTER PROTEINE
PRODUCTION DE PROTEINES CAPTIVES

(30) Priority: 09.02.2004 US 543324 P
(43) Date of publication of application: 22.11.2006
(73) Proprietor: Synamem Corporation, Burlingame, CA 94010 (US)
(72) Inventor: YAMAZAKI, Victoria, San Francisco, CA 94107 (US); SIRENKO, Oksana, San Mateo, CA 94403 (US); GROVES, John, T., Mountain View, CA 94043 (US)
(74) Representative: Williams, Paul Edwin
(86) International application number: PCT/US2005/004497
(87) International publication number: WO 2005/078104

(56) References cited:
- WO-A-89/01041
- WO-A-94/18317
- WO-A-96/41865
- WO-A-03/089649
- WO-A1-90/12099
- WO-A1-92/01715
- B.G. WERNEBURG ET AL.: "Molecular characterization of CD40 signaling intermediates" J. BIOL. CHEM., vol. 276, no. 46, 16 November 2001 (2001-11-16), pages 43334-43342,

### SEQUENCE LISTING

<110> Synamem Corporation
   Yamazaki, Victoria
   Sirenko, Oksana
   Groves, John T.
<120> Method for Generating Tethered Proteins
<130> 547958003WO0
<140> Not Yet Assigned
   <141> Filed Herewith
<150> US 60/543,324
   <151> 2004-02-09
<160> 5
<170> FastSEQ for Windows Version 4.0
<210> 1
   <211> 21
   <212> PRT
   <213> Artificial Sequence
<220>
   <223> IgK leader sequence
<400> 1
   Met Glu Thr Asp Thr Leu Leu Leu Trp Val Leu Leu Leu Trp Val Pro
   1 5 10 15
   Gly Ser Thr Gly Asp
   20
<210> 2
   <211> 6
   <212> PRT
   <213> Artificial Sequence
<220>
   <223> hexa-histidine tag
<400> 2
   His His His His His His
   1 5
<210> 3
   <211> 32
   <212> PRT
   <213> Artificial Sequence
<220>
   <223> GPI sequence
<400> 3
   Thr Thr Asp Ala Ala His Pro Gly Arg Ser Val Val Pro Ala Leu Leu
   1 5 10 15
   Pro Leu Leu Ala Gly Thr Leu Leu Leu Leu Glu Thr Ala Thr Ala Pro
   20 25 30
<210> 4
   <211> 10
   <212> PRT
   <213> Artificial Sequence
<220>
   <223> c-Src myristoylation sequence
<400> 4
   Met Gly Ser Ser Lys Ser Lys Pro Lys Asp
   1 5 10
<210> 5
   <211> 5
   <212> PRT
   <213> Artificial Sequence
<220>
   <223> enterkinase-encoding cleavage site
<400> 5
   Asp Asp Asp Asp Lys
    1               5

## Description

### Field of the Invention

The present invention relates to a method of generating tethered extracellular or intracellular domains of transmembrane proteins using recombinant vectors.

### Background of the Invention

The vast majority of therapeutics currently on the market target components of the cell membrane. A major challenge in discovering drugs against cell membrane targets is ensuring that isolated membrane samples and their components function as they do *in vivo*. When removed from their native cellular environment, cell membranes frequently lose their most physiological aspect: fluidity. Fluidity is one of the central membrane properties which facilitates the dramatic spatial rearrangement of receptors and signaling molecules during innumerable biochemical processes, ranging from intercellular communication to viral infection. For example, polyvalent ligands induce co-localization of their target receptors, thus encoding collective properties that are appreciatively different from individual binding events. In many cases, the ability of target receptors to move and adopt complimentary configurations is paramount to determining the overall affinity of the molecular recognition event In G protein coupled receptor (GPCR) and integrin signaling, ligand binding triggers a conformational change in the receptor protein itself which, in turn, alters its association with other membrane signaling molecules. In each case, changes in the organization and mobility of membrane components occur in conjunction with signaling and recognition events.

In order to preserve the physiological integrity of cell membranes, most drug discovery programs have been forced to develop "live cell" assays for membrane targets. These approaches result in significant complexity and are often difficult to industrialize. Further, live cell systems make the mechanistic study of specific receptors highly problematic, as native cell membranes contain literally hundreds of unique receptors molecules.

U.S. Patent No. 6,228,326, describes a system known as the MembraneChip™. This technology solves many of the above-described problems as it combines the physiological rigor of the *in vivo* environment with the high throughput requirements for industry drug screening. This technology consists of supported lipid bilayers elevated slightly above silicon substrates, and isolated from each other in discrete array corral spaces. The MembraneChip™ can display cell membranes and their molecular components in a fluid and functional state characteristic of *in vivo* systems. This feature of the technology distinguishes it from other biological array technologies and facilitates an accurate representafion of a myriad of biological functions with unprecedented fidelity.

A fusion of a phospholipid anchor domain and a polypeptide heterologous to the anchor domain donor polypeptide bas been described (U.S. Patent No. 5,109,113). Further, a fusion of a glycosylphosphatidylinositol (GPI) signal domain and a polypeptide heterologous to the GPI signal domain donor polypeptide have been described (U.S Patent No. 5,374,548) for targeting cell membrane surfaces. These fusion proteins are not targeted to the cell membrane. However, purification of these fusion proteins is problematic.

WO 03/089649 discloses an expression vector comprising an amino-terminal tag sequence and a signal sequence operably linked to a nucleotide of interest, wherein the amino-terminal tag sequence is inserted between the signal sequence and the nucleotide sequence of interest.

WO 96/41865 discloses recombinant DNA constructs comprising DNA sequences derived from sequences encoding proteins FRAP, Tor1, Tor2 and other proteins capable of binding to FKBP:rapamycin.

WO 94/18317 discloses a procedure for the regulated (inducible) dimerization or oligomerization of intracellular proteins by treating the cells or organisms that harbour the proteins with cell permeable, synthetic ligands.

WO 02/061389 discloses a method for determining the antigens encoded by a genomic or cDNA library involving the transfection of dendritic or other antigen presenting cells with DNA fragments in a vector which includes a signal peptide coding sequence and a sequence which encodes a peptide for binding to a receptor on the antigen presenting cell.

Therefore, there remains a need for new methods of generating tethered membrane proteins, especially for use with the above-described devices.

### Summary of Invention

According to an embodiment of the present invention, there is provided a method of generating tethered extracellular or intracellular domains of transmembrane proteins for display on a lipid bilayer array comprising:
(a) preparing an operable expression vector consisting of a 5' signal sequence, a purification epitope tag, a sequence coding for the extracellular domain of a membrane protein, and a 3' anchor sequence for attaching or associating the extracellular domain of the membrane protein to a lipid bilayer membrane; and transfecting mammalian cells with said expression vector to generate an anchor tethered protein targeted to the extracellular domain of a plasma membrane; or
(b) preparing an operable expression vector consisting of a 5' myristoylation encoding sequence, a sequence coding for the intracellular domain of a membrane protein, and a 3' purification epitope tag; and transfecting mammalian cells in vitro with said expression vector to generate a myristoylated tethered protein targeted to the intracellular domain of a membrane, and
(c) displaying the proteins of (a) or (b) on a lipid bilayer array or purifying and reconstituting in membranes the proteins of (a) or (b) for displaying on a lipid bilayer array.

Preferably, the 3' anchor sequence is a GPI anchor sequence.

Preferably, the GPI-anchor sequence comprises the 32 terminal amino acids of the GPI-anchoring sequence.

Preferably the mammalian cells are selected from the group consisting of CHO or HEK-293 cells.

Preferably, the signal sequence is selected from a protein selected from the group consisting of epidermal growth factor, insulin, nerve growth factor, platelet-derived growth factor, glucagon, ICAM-1, B7-1, TrkA, platelet-derived growth factor receptor, and CD58.

Preferably, the purification epitope tag is a hexa-histidine epitope tag.

Preferably, the myristoylation-encoding sequence is a c-Src myristoylation-encoding sequence.

Preferably, the method further comprises purifying the anchor tethered protein.

According to a further embodiment of the present invention, there is provided an operable expression vector for generating a tethered extracellular domain protein for display on a lipid bilayer array consisting of:
a 5' signal sequence,
a purification epitope tag;
a sequence coding for the extracellular domain of a membrane protein; and
a 3' anchor sequence for attaching or associating the extracellular domain of the membrane protein to a lipid bilayer membrane present in a lipid bilayer array.

Preferably, the anchor sequence is a GPI sequence.

Preferably, the purification epitope tag is a hexa-histidine epitope tag.

According to a further embodiment of the present invention, there is provided an expression vector for generating a tethered intracellular domain protein for display on a lipid bilayer array consisting of:
a 5' signal sequence for myristoylation;
a sequence coding for the intracellular domain of a membrane protein; and
a 3' purification epitope tag,
wherein the 5' signal sequence for myristoylation allows display of the intracellular domain of the membrane protein in a lipid bilayer array.

Preferably, the purification epitope tag is a hexa-histidine epitope tag.

### Brief Description of the Figures

Figs. 1A-1B show the expression vectors pYBS101 and pYBS201, respectively,
Figs. 2A-2C show FACS analysis of the experiment described in Example 3 using ICAM-1 (Fig. 2A) and B7-1 (Fig. 2B), as well as an SDS-PAGE and Western immunoblot analysis (Fig.2C) of ICAM-1 and B7-1;
Figs. 3A-3C are graph depicting cell adhesion to MembraneChip™-displayed proteins;
Figs. 4A-4B show the results of "immunological synapse" and T cell activation experiments to further determine the functionality of MembraneChip™-displayed proteins. Fig. 4A shows fluorescence microscopy of immature and mature synapses for murine T cells specific for the PCC antigen on MembraneChips™ displaying ICAM-1 and PCC-loaded I-E^{k}. Fig. 4B is a graph of the quantitation of the images in Fig. 4A.

### Detailed Description

### I. Definitions

"Expression vector" and "plasmid" are used and refer to single stranded or double stranded DNA molecules that can be used to carry a specific gene into a target cell. Once the expression vector in inside cell, the protein that is coded for by the gene is produced by the normal transcription and translation processes of the host cell. It will be appreciated that the DNA molecule may be operably linked to a control sequence such as a promoter. It will further be appreciated that the expression vector may include additional regulatory sequences to control transcription and/or translation.

"Extracellular domain" as used herein refers to the extracellular portion of a membrane protein. These segments generally are binding sites for physiological ligands for example, neurotransmitters and hormones. If the polypeptide chain of the protein crosses the bilayer several times, the extracellular domain can comprise several "loops" sticking out of the membrane.

"Intracellular domain" as used herein refers to the intracellular (or cytoplasmic) domain of a membrane protein.

"Purification epitope tag" as used herein refers to a peptide sequence used to purify the expressed protein from lysed cells. An exemplary purification peptide is hexa-histidine.

"Signal sequence" as used herein refers to a peptide sequence that directs the post-translational transport of a protein.

"Anchor" and "tether" as used herein refer to a sequence for attaching or associating a membrane protein domain with a lipid or lipid bilayer. Exemplary anchor sequences encode a GPI tether or a myristoylation tether.

The practice of the present invention will employ, unless otherwise indicated, conventional methods of molecular biology, chemistry, biochemistry, recombinant DNA techniques and immunology, within the skill of the art. Such techniques are explained fully in the literature. See, *e.g.*, Handbook of Experimental Immunology, Vols. I-IV (D.M. Weir and C.C. Blackwell eds., Blackwell Scientific Publications); A.L. Lehninger, Biochemistry (Worth Publishers, Inc., current addition); Sambrook, et al., Molecular Closing: A Laboratory Manual (2nd Edition, 1989); Methods In Enzymology (S. Colowick and N. Kaplan eds., Academic Press, Inc.).

Before describing the present invention in detail, it is to be understood that this invention is not limited to particular formulations or process parameters as such may, of course, vary. It is also to be understood that the terminology used herein is for the purpose of describing particular embodiments of the invention only, and is not intended to be limiting.

Although a number of methods and materials similar or equivalent to those described herein can be used in the practice of the present invention, the preferred materials and methods are described herein.

### II. Tethered Proteins

Membrane proteins constitute a significant proportion of total proteins in the human genome, and have been shown to regulate cellular processes as diverse as growth and differentiation, cell-cell adhesion, neuronal synaptogenesis, and immune cell activation. It is estimated that of the almost 20,000 well-characterized human proteins (NCBI Human Reference Protein Sequence, 9/10/03), approximately 24.3% are predicted to have transmembrane segments by the TMHMM algorithm (Technical University of Denmark). While 2,658 of these are predicted to have a single transmembrane domain, a large number of the 531 human proteins predicted to have two transmembrane domains likely span the membrane only once, as the hydrophobic nature of the signal peptides make them often register as transmembrane segments on routine database searches. Approximately 0.5% of these signal sequencing-bearing proteins are predicted to already contain a GPI anchor, a naturally occurring membrane tether. In essence, the present invention enables the use and study of over 66% of the membrane proteins, as an additional protein category including proteins with multiple transmembrane domains, but with discrete extracellular ligand binding domains and intracellular signaling domains, have not been accounted for in the above estimate. Consequently, the universal method of protein expression and display described herein facilitates discovery and research pertaining to both the extracellular and intracellular aspects of the vast majority of membrane proteins.

Transmembrane receptors are integral membrane proteins, which reside and operate typically within a cell's plasma membrane, but also in the membranes of some subcellular compartments and organelles. Binding to a signalling molecule or sometimes to a pair of such molecules on one side of the membrane, transmembrane receptors initiate a response on the other side. In this way they play a unique and important role in cellular communication and signal transduction.

Membrane proteins generally have three distinct segments: the extracellular domain, the transmembrane domain, and the intracellular domain. The extracellular domain is the part of the protein that sticks out of the membrane on the outside of the cell or the lumenal face of an intracellular organelle. If the polypeptide chain of the protein crosses the bilayer several times, the extracellular domain can comprise several "loops" sticking out of the membrane. This domain can facilitate the binding of ligands. The transmembrane domain spans the membrane. In the majority of proteins for which structural evidence exists, transmembrane alpha helices make up most of the transmembrane domain. In certain proteins, such as the nicotinic acetylcholine receptor, the transmembrane domain forms a protein-lined pore through the membrane, or ion channel. Upon activation of an extracellular domain by binding of the appropriate ligand, the pore becomes accessible to ions, which then pass through. In other proteins, the transmembrane domains are presumed to undergo a conformational change upon binding, which exerts an effect intracellularly. In some receptors, such as members of the 7TM superfamily (e.g. GPCRs), the transmembrane domain may contain the ligand binding pocket (evidence for this and for much of what else is known about this class of receptors is based in part studies of bacteriorhodopsin, the detailed structure of which has been determined by crystallography). The intracellular (or cytoplasmic) domain of the receptor interacts with the interior of the cell or organelle, relaying the signal (www.wikipedia.com).

Many transmembrane receptors are composed of two or more protein subunits which operate collectively and may dissociate when ligands bind, fall off, or at another stage of their "activation" cycles. They are often classified based on their molecular structure, or because the structure is unknown in any detail for all but a few receptor based on their hypothesized (and sometimes experimentally verified) membrane topology. The polypeptide chains of the simplest are predicted to cross the lipid bilayer only once, while others cross as many as seven times (the so-called G-protein coupled receptors) (www.wikipedia.com).

Membrane proteins have varying topologies in the membrane with the more hydrophobic portions of the protein being embedded in the membrane. The proteins may have a single membrane-spanning element with the N-terminus and the C-temninus located on opposite ends of the membrane or a N-terminal signal peptide that is cleaved generating a new N-terminus on the extracellular side of the membrane. In other embodiments, membrane proteins may have multiple transmembrane segments. Further, many membrane proteins are not embedded in the membrane but are tethered to the membrane by a covalently attached fatty acid that becomes part of the lipid bilayer (Creighton, Proteins: Structures and Molecular Properties, 2^{nd} Ed., W.H. Freeman and Company, New York, 1984).

### II. Method of Generating Tethered Proteins

In one embodiment, the present invention provides methods for preparing tethered extracellular or intracellular domains of transmembrane proteins. These tethered proteins are especially useful for displaying the extracellular and intracellular domains of transmembrane proteins by anchoring the domains to a fluid bilayer membrane, such as, but not limited to, those present in the MembraneChip™ described in U.S. Patent No. 6,228,326.

Particularly, the membrane proteins are tethered to an anchor such as, for example, a GPI anchorage, lipid attachments (e.g. myristoylation or palmitoylation), avidin/streptavidin/neutravidin protein fusions binding to biotinylated membrane lipids, hexa-histidine-tagged proteins tethered via Ni²⁺ membrane coordination, glutathione-S-transferase fusion proteins binding to membrane lipids conjugated with glutathione, and maltose-binding protein fusion proteins binding to membrane lipids conjugated with maltose.

In a preferred embodiment, the membrane proteins are genetically engineered to contain the tether linkage as will be discussed further below.

### A. Extracellular Domains

Exemplary extracellular domains of membrane proteins include human B7-1, ICAM-1, ErbB1, ErbB4, and the murine I-E^{k} dimer composed of α and β polypeptide chains.

Intercellular signaling is of fundamental importance to cancer, as tumors often require intimate contact between cells for their growth and maintenance. The single-pass erythropoietin producing hepatoma (Eph) receptors (EphRs) are the largest family of receptor tyrosine kinases (RTKs), and have been shown to be important for carcinogenesis (Dodelet et al., Oncogene (2000) 19:5614-5619). Moreover, the ligands of EphRs, known as Ephrins, are either single-pass or GPI-anchored membrane proteins (Cutforth et al., Trends Neurosci. (2002) 25:332-334), reflecting the critical role of cell-cell contact for transmembrane signal transduction by this receptor family. EphRs/Ephrin signaling has a fundamental role in the process of angiogenesis, the elaborate program by which tumors become vascularized in order maintain an oxygenated state. Angiogenesis thus requires intimate cell-cell interactions and signaling, and has also been a key mechanistic target for therapeutic intervention for cancer (Huang et al., Proc. Natl. Acad. Sci. USA 100:7785-7790). Using the methods of the present invention, the extracellular domains of the EphRs or Ephrin ligands can be displayed, and intact signal transduction pathways through their cognate receptors on overlaid intact cells can be measured. In the case of cancer, the appropriate EphR/Ephrin pairing mediating angiogenesis can be studied for purposes of identifying drugs which perturb the signaling pathway. Consequently, this method of displaying single-pass membrane proteins enables the identification and validation of novel cancer chemotherapeutics.

In addition to tissue regeneration and cancer, the process of immunological activation requires extensive intercellular interaction and signal transduction. The sequential stages which ultimately lead to cytokine release and proliferation of activated T cells necessitate intimate association with APCs. In the case of the T cell/APC interaction, the cell-cell contact is extremely tight as to be referred to in "synaptic" terms and greatly facilitates the precise patterning of T cell receptor, MHC, adhesion, and co-stimulatory molecules which mediate the immune response (Grakoui et al., Science (1999) 285:221-227). Moreover, supported lipid bilayers displaying ICAM-1 in the context of MHC and specific antigen peptide have been shown to be sufficient for immune synapse formation and bone fide activation of overlaid intact T cells (Grakoui et al., Science (1999) 285:221-227). As MHC molecules, ICAM-1, and the majority of other adhesion and co-stimulatory molecules are either single-pass or GPI-anchored membrane proteins, the present method of displaying such proteins affords a powerful opportunity for studying immune system activation. Further, as excessive T cell activation underlies a number of severely debilitating autoimmune diseases, including multiple sclerosis and Crohn's disease, this technology can lead to better treatments for these immunological disorders.

### B. Intracellular Domain

The present invention also facilitates the study of intracellular signaling through display of the cytoplasmic domains of membrane proteins. RTKs are one particular family of receptors whose intracellular signal transduction pathways are potential targets for anticancer therapy. The epidermal growth factor receptor, perhaps the most well-known RTK, is upregulated in a number of cancers (Gill et al, Mol. Cell Endocrinol. (1987) 51:169-186), and is the target for the antibody therapeutics Erbitux and Herceptin. As the cytoplasmic kinase domains of RTKs have been shown to have constitutive activity when separated from their extracellular and membrane-spanning domains, it may be possible to reconstitute the signal transduction pathway emanating from an intracellular domain tethered to a fluid bilayer, such as present on the MembraneChip^{™}. In this embodiment, the intracellular domain of an RTK is anchored to the MembraneChip^{™} through an N-terminal membrane anchor, followed by incubation with cell extracts or purified components of the signaling pathway. In this way, *in vitro* reconstitution of an RTK pathway facilitates the identification of small molecule drugs which can be screened for incredible specificity. Various RTKs can be arrayed on the MembraneChip^{™} in the presence of common signaling machinery, allowing for identification of inhibitors which are specific for a given RTK. Target RTKs are arrayed in parallel with non-target RTKs, enabling the identification of highly-specific small molecule drugs affecting tyrosine kinase pathways and possibly active against various cancers.

In a preferred embodiment, the intracellular domain of interest in the RTK proteins is the tyrosine kinase domain. Further suitable intracellular domains may or may not have tyrosine kinase activity, depending upon their mode of biological activity, and are selected from integrins and receptors such as tumor necrosis factor receptor, epidermal growth factor receptor, and interferon receptors. It will be appreciated that many intracellular domains are known in the art and are suitable for use in the invention.

### C. Expression Vectors

In a preferred embodiment, the tethered membrane proteins are genetically engineered using expression vectors constructed as described further below. Incorporation of a tether sequence, such as a GPI attachment signal, into a gene causes the protein to be post-translationally modified by the cell resulting in the tethered protein. For example, where a GPI attachment signal is used, a GPI linkage results at the signal position.

In one embodiment, the membrane protein comprises the extracellular domain of a membrane protein. The extracellular domain serves as a binding site for ligands.

In another embodiment, the membrane protein comprises the intracellular domain of a membrane protein. The intracellular domain serves as a binding site for cytoplasmic proteins (e.g. adaptors, kinases, phosphatases), i.e. for the study of the signaling pathway.

The membrane protein is first amplified by PCR by methods known in the art. For the extracellular domains of transmembrane proteins, cDNAs encoding the extracellular domain of interest are then cloned into the flexible multiple cloning site of a suitable expression vector to create pYBS 101. Suitable expression vectors are known in the art and are commercially available (Invitrogen). Methods for preparation and insertion of DNA into an expression vector are well known in the art (Sambrook, 1989). It will be appreciated that the commercial expression vector may include regulatory sequences as well as restriction sites. It will be appreciated that the expression vector may further include a selectable marker such as those that confer antibiotic resistance. In one embodiment, the selectable marker is neomycin resistance.

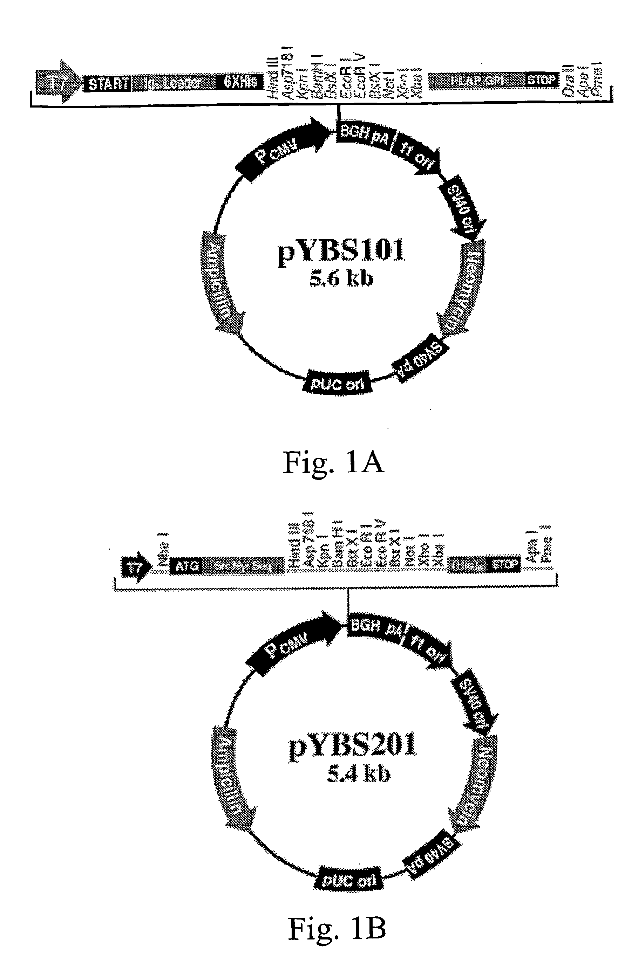

As seen in Fig. 1A, the pYBS101 vector comprises a 5' signal sequence, a purification epitope tag, and a 3' tether anchor sequence. The signal sequence targets the expressed protein to the cell surface. Suitable signal sequences are known in the art. In one embodiment, the signal sequence is the signal sequence from the Igκ chain given that it is very well-characterized. Exemplary signal sequences include nearly every secreted protein, and many membrane proteins, which are suitable for use in the invention. Examples of secreted and membrane proteins containing signal sequences include epidermal growth factor (secreted), insulin (secreted), nerve growth factor (secreted), platelet-derived growth factor (secreted), glucagon (secreted), ICAM-1 (membrane protein), B7-1 (membrane protein), TrkA (membrane protein), platelet-derived growth factor receptor (membrane protein), and CD58 (membrane protein). It will be appreciated, as known in the art, that as the genetic code is degenerate, more than one codon may encode a particular amino acid.

In a preferred embodiment, the purification epitope tag is hexa-histidine. It will be appreciated, however, that epitope tags other than hexa-histidine may be used, such as myc, Flag, HA, gluthathione-S-transferase, and maltose-binding protein.

In a preferred embodiment, the tether sequence is a GPI tether sequence. It will be appreciated that, in other embodiments, the tether is formed of avidin/streptavidin/neutravidin protein fusions binding to biotinylated membrane lipids, hexa-histidine-tagged proteins tethered via Ni²⁺ membrane coordination, glutathione-S-transferase fusion proteins binding to membrane lipids conjugated with glutathione, and maltose-binding protein fusion proteins binding to membrane lipids conjugated with maltose.

For the intracellular domains of a membrane protein, cDNAs encoding the intracellular domain are cloned into the flexible multiple cloning site of pYBS201. As seen in Fig. 1B, the pYBS201 vector comprises a 5' tether sequence and a 3' purification epitope tag as described above. The cDNA encoding the intracellular domains of membrane proteins are subcloned into the multiple cloning site flanked by these two modification sequences. In this embodiment, the tether sequence is preferably a myristoylation-encoding sequence. In one embodiment, the tether sequence is from the c-Src protein, however, the corresponding sequence of nearly any N-terminally myristoylated protein may be used instead. Examples of such proteins include other members of the Src family of tyrosine kinases, calcineurin B, members of the ADP-ribosylating factor family of proteins, nitric oxide synthase, and cAMP-dependent protein kinase.

Optionally, cDNAs may be subcloned with an enterokinase-cleavage site at the 5' end (for pYBS101) or 3' end (for pYBS201), providing the ability to remove the purification tag if necessary. Other proteases and protease-recognition sequences can also be used instead of enterokinase, including, but not limited to, thrombin and Factor Xa. While pYBS101 and pYBS201 are shown with neomycin resistance genes for selection of stably-transfected cells, it will be appreciated that the vectors are easily modified to enable selection in a number of other drugs including, but not limited to, hygromycin and zeocin. This added flexibility facilitates selection of transfected cells co-expressing different proteins, such as a multimeric complex.

As described in Examples 1 and 3, the plasmid pYBS101 was constructed in order to generate a membrane-tethered form of the extracellular domain of a membrane protein. In Example 3, the extracellular domain of a membrane protein was human B7-1 or ICAM-1. The pYBS101 plasmid is a versatile expression vector made by modifying pcDNA3.1 (+) (Fig. 1A) to contain a 5' signal sequence, a hexa-histadine purification epitope tag, the extracellular domain of a membrane protein of interest, and a 3' GPI-anchor sequence.

As described in Example 2, the pYBS201 plasmid is a versatile expression vector made by modifying pcDNA3.1(+) (Fig. 1B) to contain a 5' myristoylation-encoding sequence (derived from the N-terminal amino acids of c-Src) and a 3' hexa-histidine epitope tag.

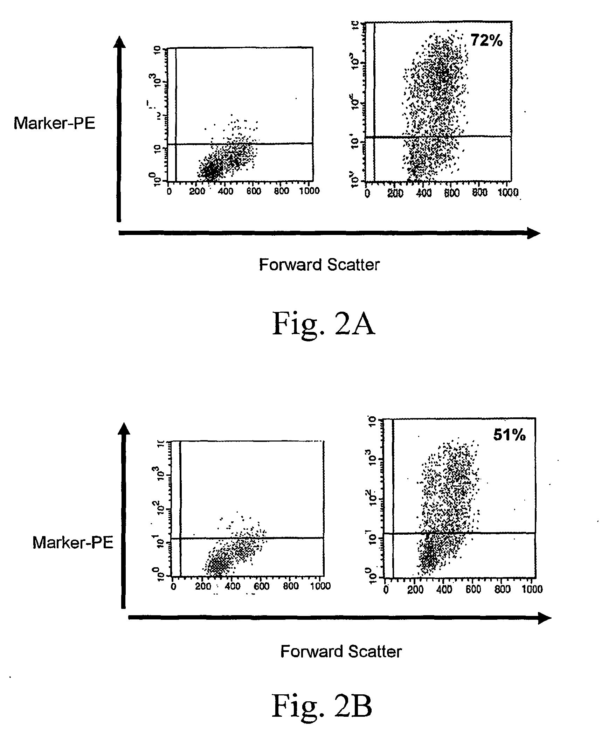

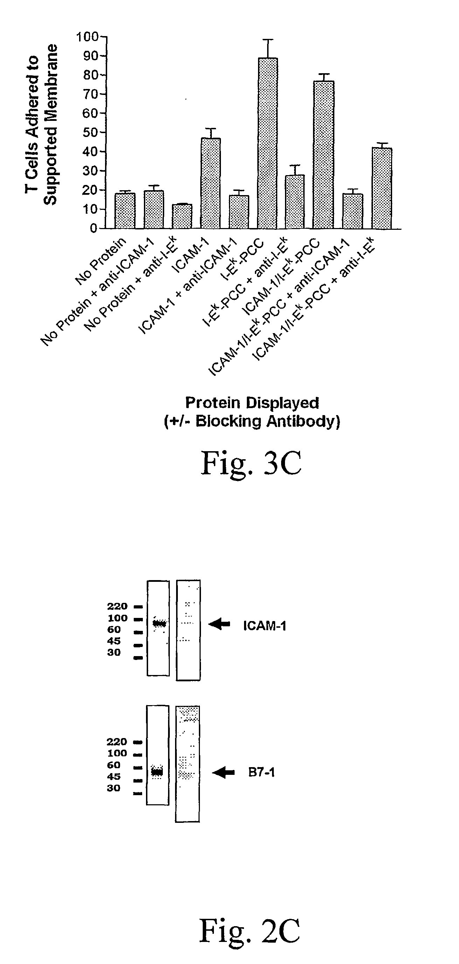

The vector is propagated by means known in the art. Expression of the domain may be evaluated by methods known in the art and exemplified by immunohistochemical staining, Western blot, and ELISA. Mammalian cells are then transfected with the vector according to known methods in the art. As seen in Example 3, transfection of the resulting pYBS101 construct(s) into mammalian cells such as Chinese hamster ovary (CHO) or HEK-293 generates an extracellular domain targeted to the plasma membrane and tethered by a C-terminal GPI anchor. It will be appreciated that transfection with pYBS201 results in an intracellular domain targeted to the plasma membrane and tethered by an N-terminal myristoylation anchor. Expression in mammalian cells can also be accomplished using retroviral vectors, depending upon how successful the standard transfection reagents are for any given protein. The fusion of an N-terminal hexa-histidine sequence facilitates rapid and high-efficiency purification on Ni²⁺ resins. Transfection efficiencies of 50-75% have been observed using methods of this invention. In some embodiments, at least 50% transfection efficiency is achieved with the present invention. In other embodiments at least 70-75% transfection efficiency is achieved with the present invention. As seen in Fig. 2A, expression of ICAM-1 in HEK-293 cells was 72%. Fig. 2A shows the results of FACS analysis using specific PE-conjugated antibodies against human ICAM-1. The left panel demonstrates essentially no expression of ICAM-1 in control untransfected cells, whereas robust expression is observed in cells transfected with human ICAM-1 in pYBS 101 (right panel). Similar results are demonstrated for expression of human B7-1 using FACS analysis in Fig. 2B. Expression of the tethered proteins can be confirmed by both SDS-PAGE and western immunoblot assay as shown in Fig. 2C. The figure shows bands corresponding to ICAM-1 or B7-1 protein (left) or immunoreactivity (right), respectively. With reference to Examples 3-11, although the examples herein relate to the generation and display of the extracellular domains of membrane proteins using pYBS101, it will be appreciated that the examples are equally applicable to the incorporation of intracellular domains of membrane proteins using pYBS201.

The purification epitope tag is used to purify the post-translational membrane protein. Expression in mammalian cells generates an extracellular or intracellular domain, respectively, tethered to the membrane by the tether which is easily purified by the Ni²⁺ resins. As described in Example 4, the epitope tag binds to beads or resins according to known methods.

### IV. Array

As discussed above, the tethered proteins formed by the methods described herein are useful for generating and displaying extracellular and intracellular domains of transmembrane proteins on lipid bilayer arrays. The extracellular and intracellular domains may be used as receptors or ligand binding domains for detecting an analyte in the array. Particularly preferred is the use of the present invention with the fluid bilayer membranes described in U.S. Patent No. 6,228,326. The ability of this fluid bilayer membrane, hereinafter referred to as MembraneChip^{™}, described therein to display characteristics of living cell membranes greatly facilitates its use for the study of membrane proteins which regulate nearly every aspect of cellular physiology.

These membrane arrays find use in applications including simple quality assurance/quality control optimization studies, medical diagnostics, and drug discovery involving cell-cell interactions. Intracellular domains displayed using these methods enable investigation of whole intact signal transduction pathways. The method of display described herein, which can handle the majority of membrane proteins in the genome containing a discrete functional intracellular or extracellular domain, enables a myriad of applications in both industry and the medical clinic.

For example, receptor tyrosine kinases (RTKs), which are critical mediators of cellular growth and differentiation and have been associated with various cancers, are greatly amenable to targeting using the present technology. The ligand-induced dimerization and autophosphorylation characteristic of RTKs can faithfully be reconstituted for drug discovery given the fluidity of the MembraneChip™ environment. In addition to studying membrane proteins regulating cell autonomous events, the technology is useful for modeling intercellular interactions, including cell-cell adhesion and trans-cellular signal transduction. Cell-cell signaling is critical for regulating cellular proliferation and differentiation, the development of organs and tissues during embryogenesis, the establishment of neuronal synapses, vasculogenesis, and the activation of an immunological response. Fundamentally, these intercellular interactions occur through the binding of cognate membrane-bound ligands and receptors, initiating signal transduction cascades in the apposing cells. Inappropriate cell-cell communication has been implicated in a number of disease states, including cancer and autoimmunity, making the associated signaling molecules important targets for drug discovery efforts. Arraying a membrane-bound ligand in a chip corral and overlaying with an intact cell expressing its membrane-bound receptor combines the advantages of having a well-defined, purified component and being able to study a complete signal transduction pathway simultaneously.

In the context of the immune system, for example, displaying antigen peptide complexed to MHC and accessory adhesion and co-stimulatory molecules in a fluid bilayer can successfully recapitulate the activity of antigen-presenting cells (APCs). Overlaying intact T cells specific for the antigen peptide displayed on the chip enables their rapid activation, facilitating the targeting of nearly every checkpoint in the T cell activation pathway (see, e.g., Grakoui et al., Science (1999) 285:221-227, for a discussion of this interaction).

The physiological qualities of the MembraneChip^{™} make it extremely useful for studying processes requiring intercellular contact. For example, GPI-linked extracellular domains of single-pass membrane proteins displayed in the supported lipid bilayer can serve as adhesion molecules and signaling ligands for cognate receptors in overlaid intact cells, enabling growth of various tissues. Numerous studies have suggested that stem cells can differentiate into various cell types *in vitro* through co-culturing with specific tissues (Prockop et al., Proc. Natl. Acad. Sci. USA (2003) 100:11917-11923). Accordingly, the membrane arrays of the present invention can facilitate the generation of organ tissue on an industrial scale. Thus, by providing minimal signals required for differentiation of stem cells into a particular tissue present in the extracellular domains of membrane proteins, stem cells can be cultured in the context of a MembraneChip™ displaying the extracellular domains of these proteins. Indeed, growing human tissue which is suitable for transplantation or implantation into a diseased organ would be a major advance in public health, particularly given that approximately 73,000 people nationally are currently awaiting an organ transplant and 10,000 die annually due to the shortage of cadaveric organs.

The array is also particularly enabling for medical diagnostics. Infectious disease is becoming an increasingly important branch of medicine, and the ability to monitor the effectiveness of vaccinations against various pathogens has become extremely valuable. Commonly, vaccination results in the production of T cells specific for the particular antigen being administered. By displaying an MHC molecule complexed with a pathogenic antigen, it is possible to isolate T cells specific for that antigen from a patient's blood serum. In this embodiment, a patient vaccinated against a whole panel of pathogens supplies a simple blood sample which is mixed with the same panel displayed in MHC on the fluid bilayer array, facilitating a single high-information content screen for responding T cells indicative of a successful vaccination. Displaying MHCs with antigen is also beneficial for diagnosing various autoimmune diseases. For example, self-antigens which have been implicated in multiple sclerosis can be displayed on a chip in order to test for the presence of the specific population of T cells thought to be responsible for the disease pathology.

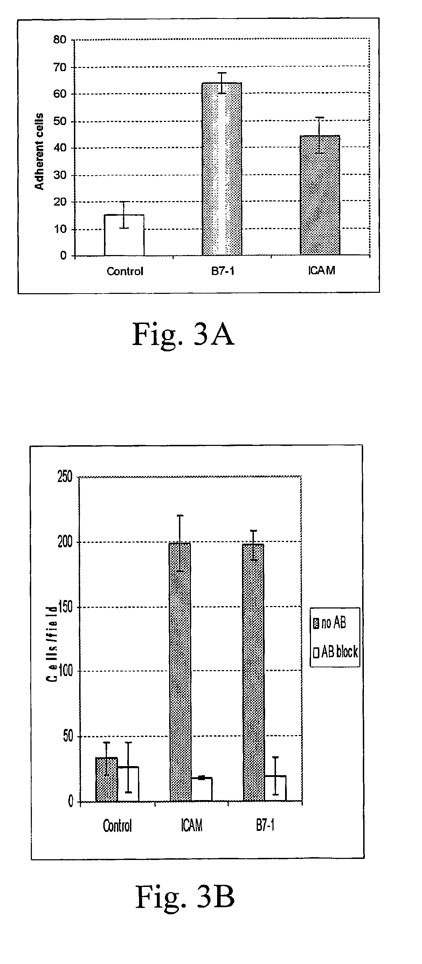

As demonstrated in Example 10, Jurkat T cells selectively adhere to bilayer membranes including tethered proteins. As seen in Figure 3A, at least 4X more Jurkat T cells adhered to MembraneChips™ displaying human B7-1 proteins than the control. For MembraneChips™ displaying human ICAM proteins, at least 3X more adhesion was observed. Further, adhesion of Jurkat T cells was inhibited by pre-incubating the MembraneChip™ with monoclonal antibodies against the extracellular domains of human B7-1 and ICAM-1 (Fig. 3B), respectively, showing the specificity of adhesion for the displayed proteins. Fig. 3B is a graph of Jurkat T cell adhesion for a control, membrane with tethered ICAM-1 protein, membrane with tethered B7-1 protein, with and without the antibody block. Adhesion was measured as the number of cells per field. As seen in Fig. 3B, use of the antibody block resulted in at least 8X less adhesion of the Jurkat T cells than without the antibody block.

Fig. 3C, shows antigen-specific T cell adhesion to MembraneChips™ with and without antibody blocks for ICAM-1, I-E^{k}-PCC, and an empty membrane control. In another embodiment, the bilayer may include two or more tethered proteins. As described in Example 10, MembraneChips^{™} were prepared with ICAM-1 and I-E^{k}-PCC tethered proteins. In this manner, more than one analyte may be detected with the same MembraneChip™ composition. Both proteins remained functional. As seen in Fig. 3C, about half of the number of cells adhered to the ICAM-1/IEk-PCC after pretreatment with anti-I-E^{k} antibodies as compared to the MembraneChip™ without pretreatment with the monoclonal antibody.

The present method also provides a powerful device for quality assurance and control applications. Drugs (including but not limited to small molecules, antibodies, and biologicals) which have been identified to have activity against any of the membrane proteins displayed using this method could easily be characterized and optimized using this technology. The array format of the technology easily facilitates studies of specificity and sensitivity, and is compatible with a number of different detection systems, including the label-free approach as described in U.S. Patent Publication No. 20040053337 and surface plasmon resonance. In this manner, affinity measurements for active drugs are obtained very rapidly, facilitating further optimization and validation. Consequently, the present method of membrane protein display is extremely advantageous for a number of different fields.

**Table 1: Immunological Membrane Proteins**

| Single-Pass Membrane Proteins in the Immunological Synapse | | |
|---|---|---|
| | APC | T Cell |
| Signaling Molecules | MHC I | TCR |
| | MHC II | CD4 |
| | | CD8 |
| | | CD3 |
| Co-stimulatory Molecules | B7-1 (CD80) | CD40L |
| | B7-2 (CD86) | CD28 |
| | ICOSL | ICOS |
| | CD40 | PD-L1 |
| | PD-1 | PD-L2 |
| | | 4-1BB (CD137) |
| Adhesion Molecules | ICAM-1 | LFA1 |
| | LFA3 (CD58) | CD2 |

The membrane proteins are tethered to lipids, which are then used to form a fluid bilayer as described in U.S. Patent No. 6,228,326. The membrane proteins produced by the expression vectors described above are tethered to the lipids of a bilayer membrane for use with the bilayer array. The purified proteins are added to a mixture of vesicle-forming lipids and dialyzed to form a vesicle suspension. The lipid used is dependent upon the characteristics needed for the bilayer. Exemplary lipids include synthetic and naturally-occurring lipids including the phospholipids such as phosphatidylcholine (PC), phosphatidylethanolamine (PE), phosphatidylserine (PS), etc. The lipid-protein vesicles are then deposited onto a substrate for forming the bilayer array. The substrate includes at least one bilayer compatible surface region. Typically, the substrate surface is contacted with the lipid-protein vesicle suspension. The suspension is allowed to remain in contact with the surface for a time, suitable for the lipid to fuse with the surface and form a fluid bilayer. The time required is typically several minutes, e.g. about 5 minutes.

As described in Example 5, purified proteins were added to an egg PC lipid mixture to make a vesicle suspension. The vesicle suspension was deposited and incubated with MembraneChips^{™} or cover slips to from fluid bilayers with tethered membrane proteins. In corporation of the protein into the bilayer membrane was confirmed by immunofluorescence. Cell adhesion to the displayed domains can be confirmed by any means known in the art, and as described in Examples 6-7. Validation of the membrane protein display was confirmed using immunofluorescence microscopy as described in Example 9.

While the T cell adhesion experiments depicted in Example 10, and shown in Figs. 3A-3C, demonstrate the activity of membrane proteins expressed, purified, and displayed using the methods of the present invention, the examination of immune synapse formation demonstrates the fluidity and signaling capacity of then proteins in the lipid bilayer of the MembraneChip™. Signal transduction pathways often require oligomerization and/or clustering of cell surface molecules in order to transmit signals across the plasma membrane of cells, a necessary phenomenon made possible by lateral fluidity of physiological membranes. As described in U.S. Patent Nos. 6,228,326 and 6,503,452; U.S. Patent Publication Nos. 20020009807 and 20020160505; and PCT Publication No. WO 01/26800, MembraneChip™ supported lipid bilayers exhibit a physiological level of fluidity. Mobility of membrane proteins generated by the methods described here were examined in the context of "immunological synapse" formation, a dynamic stereotyped process of T cell activation whereby T cell receptot/MHC and LFA-1/ICAM-1 pairs undergo a dramatic reorganization at the cell surface (Grakoui et al., Science (1999) 285:221-227).

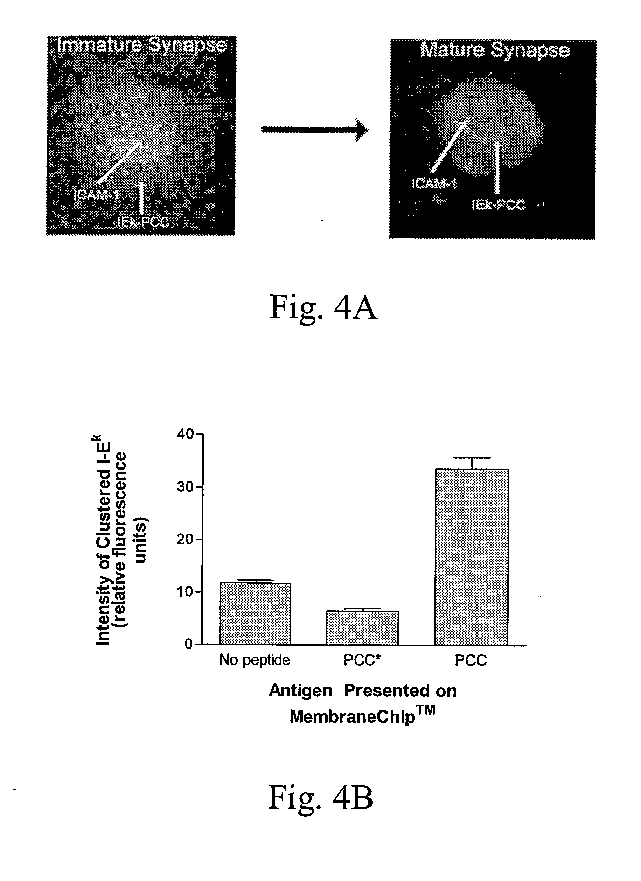

As described in Example 8, domains that are displayed on the MembraneChip™ retain the function of the domain. Fig. 4A shows the immature and mature synapse of ICAM-1 and I-E^{k}-PCC loaded MembraneChips™ when contacted with PCC-specific T cells. Upon initial encounter of the PCC-specific murine T cells with MembraneChips™ displaying ICAM-1 (designated with the arrow labeled ICAM-1) and PCC-loaded I-E^{k} (designated with the arrow labeled IEk-PCC), T cells adhere and form an immature "synapse" by ICAM-1 localized centrally and I-E^{k} at the periphery (Fig. 4A, left panel). During the next 30 minutes ofT cell engagement, however, this "synaptic" pattern changes dynamically, as the PCC-loaded I-E^{k} migrates centrally and ICAM-adopts a more peripheral localization (Fig. 4A, right panel). This dramatic rearrangement of ICAM-1 and I-E^{k} is entirely antigen-dependent, as it does not occur in the context of an empty I-E^{k} or an I-E^{k} loaded with a mutated PCC* peptide (data not shown). Moreover, relocalization of these proteins following engagement with live T cells underscores the physiological level of lateral fluidity which these tethered proteins exhibit. In addition to the qualitative assessment of immune synapse formation, more quantitative information is readily obtained by measuring the extent of I-E^{k} clustering at the center of the T cell/APC synapse. As depicted in Fig. 4B, engagement of MembranChips™ displaying ICAM-1 and PCC-loaded I-E^{k} leads to an approximately 3-4-fold increase in clustered I-E^{k} molecules in comparison to membranes displaying empty I-E^{k} or I-E^{k} complexed to the mutated PCC* peptide.

### EXAMPLES

Efforts have been made to ensure accuracy with respect to numbers used (e.g., amounts, temperatures, etc.), but some experimental error and deviation should, of course, be allowed for.

### Materials and Methods

### Cells:

CHO, HEK-293, and Jurkat T cells were obtained from the University of California, San Francisco Cell Culture Facility and cultured in Ham's F12, Dulbecco's Modified Eagle Medium, and RPMI 1640 media, respectively, in 37°C humidified incubators with 5% CO₂, according to manufacturer's recommendations.

### Example 1

### Generation of Expression Vector Producing GPI tethered Extracellular Domain

pYBS 101 was generated through modification of pcDNA3.1 (+) (Invitrogen). The sequence encoding the Igκ leader sequence (SEQ ID NO: 1) followed by a sequence encoding six tandem histidine residues (SEQ ID NO:2) was cloned into *NheI-Hind*III-digested pcDNA3.1(+) as an oligonucleotide, regenerating only the *Hind*III site. The sequence encoding the 32 terminal amino acids of the GPI-anchoring sequence (SEQ ID NO:3) from placental alkaline phosphatase was PCR amplified from pVac2-mcs (Invivogen) as an *Xba*I*-Nhe*I fragment and ligated into the *Xba*I site of pcDNA3.1 (+) with the previously mentioned modification, regenerating the *Xba*I site only at the 5' end of the sequence, generating pYBS101.

### Example 2

### Generation of Expression Vector Producing Myristoylation Tethered Intracellular Domain

pYBS201 was generated through modification of pcDNA3.1 (+) (Invitrogen). The sequence encoding the c-Src N-terminal myristoylation sequence (SEQ ID NO:4) was cloned into *Nhe*I*-HindIII-digested* pcDNA3.1(+) as an oligonucleotide. The sequence encoding six tandem histidine residues (SEQ ID NO:2) was subsequently cloned into the XbaI-ApaI-digested vector containing the myristoylation-encoding sequence as an oligonucleotide. The result was pYBS201, containing both a 5' c-Src myristoylation- and a 3' hexa-histidine-encoding sequence.

### Example 3

### Expression of Membrane Proteins in GPI-anchored form

cDNAs encoding the extracellular domains (excluding signal sequences) of human B7-1 and ICAM-1 were amplified from ESTs (Invitrogen) and cloned into pYBS 101 as *Hind*III*-Xba*I fragments with enterokinase-encoding cleavage sites (DDDDK, SEQ ID NO: 5) at their N-termini. CHO cells were transfected with the expression plasmids using calcium phosphate transfection (Promega) or SuperFect (Qiagen) following manufacturers recommendations. 48 hours after transfection, cells were split at 1:5, 1:10 and 1:20 into growth media containing 500 µg/ml of G418 (Invitrogen) for generation of stable lines expressing human B7-1 and ICAM-1. Polyclonal stable cell lines were maintained and passaged in G418-containing media for 4 weeks. ICAM-1 and B7-1 were also expressed transiently in HEK-293 cells. Expression of these proteins was confirmed by FACS analysis using PE-conjugated antibodies (Beckton Dickinson) and the results are shown in Figs. 2A and 2B, respectively. Expression of ICAM-1 and B7-1 was further confirmed by Western immunoblot analysis and the results are shown in Fig. 2C.

### Example 4

### Purification of Membrane Proteins

pYBS101 plasmids expressing B7-1 or ICAM-1 were prepared and CHO cells were transfected as described in Example 3. Confluent CHO cells from 6-8 10 cm dishes were washed 2X with cold PBS, and were scraped into 600 µl (per dish) of lysis buffer containing 50 mM Tris-Cl pH 8.0, 150 mM NaCl, and 1 % Triton X-100, and a protease inhibitor cocktail (Sigma). Harvested cells were solubilized on ice for 30 minutes, followed by microcentrifugation for 15 min at highest speed. Ni-NTA agarose (Qiagen) was washed 1X with lysis buffer and added to clarified extracts, 50 µL of beads per 600 µL of cell extract. Cell extracts were incubated with beads for 2 hours with rotation at 4 °C. Beads were washed 4X with buffer containing 50 mM Tris pH 8.0, 150 mM NaCl, 10 mM imidazole (Sigma), and 1 % Octyl-Glucopyranoside (Calbiochem) plus a protease inhibitor cocktail. Bound proteins were eluted with 600 µl of buffer containing 50 mM Tris pH 8.0, 150 mM NaCl, 200 mM imidazole, and 1% Octyl-Glucopyranoside plus a protease inhibitor cocktail for 1 hour at 4 °C with rotation. Eluted proteins were approximately 50% pure as examined by SDS-PAGE and was approximately 6.8 µg B7-1/mg total protein and 3.3 µg ICAM-1/mg total protein according to Bradford analysis (BioRad). Approximately, 10-50% of purified protein remained bound to the beads following elution.

### Example 5

### Reconstitution of Membrane Proteins

Aliquots of protein extracts prepared essentially by the methods of Example 4 were added to an eggPC lipid mix (1% NBD-PG, 99% eggPC) and dialyzed overnight at room temperature against PBS, with 3X buffer changes. While eggPC was used to generate membranes in this case, these proteins could easily be reconstituted in vesicles with various lipid compositions. To form the supported lipid membranes, 20 µL of undiluted (or diluted 1:2) protein-lipid mix was deposited onto MembraneChips^{™} or cover slips for 3-5 min at room temperature, followed by 5 washes with buffer. Incorporation of protein into the supported membrane was confirmed by immunofluorescence using antibodies to the respective proteins. Membranes were stained with 30 µl of PE-conjugated antibodies (Becton Dickinson) in 5% FCS for 30 min then washed extensively with PBS and assessed by fluorescent microscopy. Protein densities are approximately 10,000-20,000 molecules/µm², as estimated from a proteolipid concentration of 0.6 µg/µL (50% pure) in a 20-µL drop on a chip surface of 7 mm diameter. It was assumed that only 1 % of the drop contents were retained on the chip surface for membrane display.

### Example 6

### Jurkat T Cell Adhesion to MembraneChip™

Cell adhesion assays were performed as described by Chan et al. (Chan et al., J. Cell. Biol. (1991) 115:245-255) with several modifications. Briefly, membranes formed with aliquots of B7-1 and ICAM-1 substantially by the method of Example 5 were blocked with 10% fetal bovine serum (VWR) in PBS for 1 hour at room temperature, followed by incubation for 10 minutes with RPMI 1640 media (Invitrogen) containing 10% FBS at 37 °C. 1 × 10⁵ Jurkat T cells were added to the well for 15 minutes. The approximate number of cells per membrane corral was determined by counting under brightfield microscopy (usually 150-200 cells/corral). MembraneChips™ were subsequently inverted into a dish containing 10% FBS in PBS for 15-20 minutes to wash unbound cells. The final cell number was determined by counting under microscope directly or after taking images. In experiments where monoclonal antibodies against human B7-1 or ICAM-1 (Santa Cruz) were used to block Jurkat T cell adhesion, MembraneChips™ were pre-incubated with antibody followed by 3X washes with PBS prior to incubation with cells. Imaging was performed on a Nikon Eclipse vertical microscope.

### Example 7

### Antigen-specific T Cell Adhesion to MembraneChip™

In order to perform antigen-specific T cell adhesion, the murine class II MHC dimeric molecule I-E^{k} was expressed, purified, and displayed in the MembraneChip™ using the methods as described in Examples 3 and 4. In various experiments, I-E^{k} was displayed in the presence or absence of ICAM-1. I-E^{k} was loaded with a short peptide derived from pigeon cytochrome c (PCC), a protein antigen recognized specifically by T cells derived from the transgenic mouse strains B10.Cg-Tg(TcrAND)53Hed/J and B6;SJL-Tg(TcrAND)53Hed/JCell (The Jackson Laboratory) (Kaye et al., Nature (1989) 341: 746-749). Murine T cells specific for PCC were isolated using magnetic bead selection (Dynal) from spleens harvested from these aforementioned transgenic mice. Measurement of these murine PCC-specific T cells to MembraneChips™ displaying either ICAM-1 and PCC-loaded I-E^{k} alone or in combination was performed essentially as described for the Jurkat T cell experiments (Example 6). Monoclonal antibodies directed against human ICAM-1 and murine I-E^{k} (Santa Cruz) were used for the blocking experiments.

### Example 8

### Antigen-specific Immune Synapse formation on the MembraneChip™

Immune synapse experiments utilizing murine T cells specific for the PCC antigen and MembraneChips™ displaying ICAM-1 and PCC-loaded I-E^{k} prepared essentially as described in Example 7 (see also Grakoui et al., Science (1999) 285:221-227). Human ICAM-1 (fluorescently-labeled green) and murine I-E^{k} (fluorescently-labeled red) were expressed, purified, and displayed utilizing the methods essentially as described in Examples 3-5. Fig. 4A shows the immature synapse (left panel) of the adhesion of the murine T cells specific for the PCC antigen to the MembraneChip™ displaying ICAM-1 centrally and PCC-loaded I-E^{k} around the perimeter. The right panel shows the mature synapse after incubation for 30 minutes.

The strength of immune synapse formation was determined by the extent of central I-E^{k} clustering as determined by quantitation of images in Adobe Photoshop and the results are shown in Fig. 4B. The intensity of the clustered I-E^{k} was measured in relative fluorescence units for MembraneChips™ prepared with no peptide, mutated PCC* and wild-type PCC.

### Example 9

### Validation of Membrane Protein Display

The pYBS101 expression system was validated using several proteins of the immunological synapse which mediate the interaction of T cells with antigen-presenting cells: B7-1, ICAM-1, and the dimer I-E^{k} formed by α and β chains. cDNAs encoding the human (ICAM-1, B7-1) or murine (I-E^{k}) isoforms of these proteins were cloned into pYBS101 and expressed either stably (in CHO cells) or transiently (in HEK-293 cells) by the method of Example 3. FACS analysis (Fig. 2A and 2B) demonstrated a high level of expression of these proteins (shown for ICAM-1 and B7-1), as well as their correct targeting and tethering to the plasma membrane (untransfected on left, transfected on right). Purification of these proteins was accomplished in one step using Ni²⁺ resins as described in Example 4, and SMPAGE and Western immunoblotting indicate that these proteins were of the predicted size (Fig. 2C). The purified proteins were subsequently into egg phosphatidylcholine vesicles and arrayed into membrane corrals of the MembraneChip™ as described in Example 5.

Immunofluorescence microscopy revealed the presence of these proteins in the supported bilayers (data not shown). In cases where it would be desirable to release soluble protein from the chip array, enzymatic cleavage of the GPI anchor with phosphatidylinositol phospholipase C can be accomplished.

### Example 10

### Jurkat T Cell Adhesion Assay

In order to demonstrate that the purified, MembraneChip™-displayed proteins were functional, the ability of the system to mediate Jurkat T cell adhesion was tested (Fig. 3A). Approximately 1 × 10⁵ Jurkat T cells were incubated with MembraneChips™ with or without displayed human B7-1 or ICAM-1 prepared as described in Example 5 at densities of approximately 10,000-20,000 molecules/µm². Robust Jurkat T cell adhesion was observed only to membranes displaying these proteins, as the cells did not adhere nearly as well to membranes devoid of protein. As a further test of the specificity of the cell adhesion, the CD28B7-1 and LPA-1/ICAM-1 interaction was blocked with monoclonal antibodies against the extracellular domains of human B7-1 and ICAM-1, respectively. The result depicted in Fig. 3B demonstrate that pre-incubating membranes with antibodies against B7-1 or ICAM-1 abolished Jurkat T cell adhesion to MembraneChips™ displaying the respective proteins, reducing the cell adhesion to background levels observed in membranes devoid of protein. Moreover, murine T cells specific for the PCC antigen were examined for their adhesion to MembraneChips™ displacing ICAM-1 and PCC-loaded I-E^{k}, either alone or in combination (Fig. 3C). As in the case with the Jurkat T cell experiments, specific blockage ofT cell adhesion could be accomplished using monoclonal antibodies against ICAM-1 and I-E^{k} (Fig. 3C).

Thus, novel methods of producing tethered extracellular and intracellular domains, especially for use with fluid bilayer membranes, are described. The experiments described herein demonstrate robust activity of the tethered proteins in the context of biological assays.

## Claims

1. A method of generating tethered extracellular or intracellular domains of transmembrane proteins for display on a lipid bilayer array comprising:
(a) preparing an operable expression vector consisting of a 5' signal sequence, a purification epitope tag, a sequence coding for the extracellular domain of a membrane protein, and a 3' anchor sequence for attaching or associating the extracellular domain of the membrane protein to a lipid bilayer membrane; and
transfecting mammalian cells in vitro with said expression vector to generate an anchor tethered protein targeted to the extracellular domain of a plasma membrane; or
(b) preparing an operable expression vector consisting of a 5' myristoylation encoding sequence, a sequence coding for the intracellular domain of a membrane protein, and a 3' purification epitope tag; and
transfecting mammalian cells in vitro with said expression vector to generate a myristoylated tethered protein targeted to the intracellular domain of a membrane, and
(c) displaying the proteins of (a) or (b) on a lipid bilayer array or purifying and reconstituting in membranes the proteins of (a) or (b) for displaying on a lipid bilayer array.

2. The method according to claim 1, wherein said 3' anchor sequence is a Glycosylphosphatidylinositol (GPI) anchor sequence.

3. The method according to claim 2, wherein said GPI-anchor sequence comprises the 32 terminal amino acids of the GPI-anchoring sequence.

4. The method according to any previous claim, wherein said mammalian cells are selected from the group consisting of CHO or HEK-293 cells.

5. The method according any previous claim, wherein said signal sequence is selected from a protein selected from the group consisting of epidermal growth factor, insulin, nerve growth factor, platelet-derived growth factor, glucagon, ICAM-1, B7-1, TrkA, platelet- derived growth factor receptor, and CD58.

6. The method according to any previous claim, wherein said purification epitope tag is a hexa-histidine epitope tag.

7. The method according to any previous claim, wherein said myristoylation-encoding sequence is a c-Src myristoylation-encoding sequence.

8. An operable expression vector for generating a tethered extracellular domain protein for display on a lipid bilayer array consisting of:
a 5' signal sequence,
a purification epitope tag;
a sequence coding for the extracellular domain of a membrane protein; and
a 3' anchor sequence for attaching or associating the extracellular domain of the membrane protein to a lipid bilayer membrane present in a lipid bilayer array.

9. The vector according to claim 8, wherein said anchor sequence is a GPI sequence.

10. The vector according to claim 8 or 9, wherein said purification epitope tag is a hexa-histidine epitope tag.

11. An expression vector for generating a tethered intracellular domain protein for display on a lipid bilayer array consisting of:
a 5' signal sequence for myristoylation;
a sequence coding for the intracellular domain of a membrane protein; and
a 3' purification epitope tag,
wherein the 5' signal sequence for myristoylation allows display of the intracellular domain of the membrane protein in a lipid bilayer array.

12. The vector according to claim 11, wherein said purification epitope tag is a hexa-histidine epitope tag.

13. The method according to any one of claims 1 to 7, further comprising: purifying said anchor tethered protein.

## Patentansprüche

1. Eine Methode zur Erzeugung von gebundenen, extrazellulären oder intrazellulären Domänen transmembranöser Proteine zum Display auf einem Lipid-Doppelschicht-Array, bestehend aus:
(a) Vorbereitung eines operablen Expressionsvektors bestehend aus einer 5'-Signalsequenz, einem Purifikations-Epitop-Tag, einer Sequenzcodierung für die extrazelluläre Domäne eines Membranproteins und einer 3'-Anker-Sequenz zwecks Verbindung oder Assoziation der extrazellulären Domäne des Membranproteins mit einer Lipid-Doppelschichtmembran; sowie
Transfektion von Säugetierzellen in vitro mit dem genannten Expressionsvektor zur Erzeugung eines an einen Anker gebundenen Proteins, das auf die extrazelluläre Domäne einer Plasmamembran abzielt; oder
(b) Vorbereitung eines operablen Expressionsvektors bestehend aus einer 5'-Myristoylations-Codiersequenz, einer Sequenzcodierung für die intrazelluläre Domäne eines Membranproteins und einem 3'-Purifikations-Epitop-Tag; sowie
Transfektion von Säugetierzellen in vitro mit dem genannten Expressionsvektor zur Erzeugung eines myristoylierten, gebundenen Proteins, das auf die intrazelluläre Domäne einer Membran abzielt, sowie
(c) Display der Proteine von (a) oder (b) auf einem Lipid-Doppelschicht-Array oder Purifikation und Rekonstitution in Membranen der Proteine von (a) oder (b) zum Display auf einem Lipid-Doppelschicht-Array.

2. Die Methode gemäß Anspruch 1, wobei die genannte 3'-Anker-Sequenz eine Glycosylphosphatidylinositol-(GPI)-Anker-Sequenz ist.

3. Die Methode gemäß Anspruch 2, wobei die genannte GPI-Anker-Sequenz die 32 Terminal-Aminosäuren der GPI-Anker-Sequenz umfasst.

4. Die Methode gemäß einem der vorhergehenden Ansprüche, wobei die genannten Säugetierzellen aus der Gruppe bestehend aus CHO- oder HEK-293-Zellen ausgewählt werden.

5. Die Methode gemäß einem der vorhergehenden Ansprüche, wobei die genannte Signalsequenz von einem Protein gewählt wird, das aus der Gruppe bestehend aus epidermalem Wachstumsfaktor, Insulin, Nervenwachstumsfaktor, Plättchen-abgeleitetem Wachstumsfaktor, Glucagon, ICAM-1, B7-1, TrkA, Plättchen-abgeleitetem Wachtsumsfaktorrezeptor und CD58 ausgewählt wird.

6. Die Methode gemäß einem der vorhergehenden Ansprüche, wobei das genannte Purifikations-Epitop-Tag ein Hexa-Histidin-Epitop-Tag ist.

7. Die Methode gemäß einem der vorhergehenden Ansprüche, wobei die genannte Myristoylations-Codier-Sequenz eine c-Src-Myristoylations-Codier-Sequenz ist.

8. Ein operabler Expressionsfaktor zur Erzeugung eines gebundenen extrazellulären Domänproteins zwecks Display auf einem Lipid-Doppelschicht-Array bestehend aus:
einer 5'-Signalsequenz,
einem Purifikation-Epitop-Tag,
einer Sequenzcodierung für die extrazelluläre Domäne eines Membranproteins; sowie
einer 3'-Anker-Sequenz zur Bindung oder Assoziation der extrazellulären Domäne des Membranproteins an eine Lipid-Doppelschichtmembran, die in einem Lipid-Doppelschicht-Array vorhanden ist.

9. Der Vektor gemäß Anspruch 8, wobei die genannte Anker-Sequenz eine GPI-Sequenz ist.

10. Der Vektor gemäß Anspruch 8 oder 9, wobei das genannte Purifikations-Epitop-Tag ein Hexa-Histidin-Epitop-Tag ist.

11. Ein Expressionsfaktor zur Erzeugung eines gebundenen intrazellulären Domänproteins zwecks Display auf einem Lipid-Doppelschicht-Array bestehend aus:
einer 5'-Signalsequenz zur Myristoylation,
einer Sequenzcodierung für die intrazelluläre Domäne eines Membranproteins; sowie
einem 3'-Purifikations-Epitop-Tag;
wobei die 5'-Signalsequenz zur Myristoylation das Display der intrazellulären Domäne des Membranproteins in einem Lipid-Doppelschicht-Array ermöglicht.

12. Der Vektor gemäß Anspruch 11, wobei das genannte Purifikations-Epitop-Tag ein Hexa-Histidin-Epitop-Tag ist.

13. Die Methode gemäß einem der Ansprüche 1 bis 7, die zudem aus der Purifikation des genannten, an den Anker gebundenen Proteins besteht.

## Revendications

1. Méthode de génération de domaines intra ou extracellulaires attachés de protéines transmembranaires en vue de leur présentation sur un réseau de bicouches lipidiques prévoyant de :
(a) prépaper un vecteur d'expression opérable consistant en une séquence signal en 5', une étiquette épitopique de purification, une séquence codant pour le domaine extracellulaire d'une protéine membranaire, et une séquence d'ancrage en 3' pour attacher ou associer le domaine extracellulaire de la protéine membranaire à une membrane à bicouche lipidique ; et
transfecter in vitro des cellules de mammifères avec ledit vecteur d'expression afin de générer une protéine d'ancrage attachée ciblant le domaine extracellulaire d'une membrane plasmatique ;
(b) préparer un vecteur d'expression opérable consistant en une séquence de codage de myristoïlation en 5', une séquence codant pour le domaine intracellulaire d'une protéine membranaire, et une étiquette épitopique de purification en 3' ; et
transfecter in vitro des cellules de mammifères avec ledit vecteur d'expression pour générer une protéine myristoïlée ciblant le domaine intracellulaire d'une membrane, et
(c) présenter des protéines de (a) ou de (b) sur un réseau de bicouches lipidiques ou purifier et reconstruire, dans des membranes, des protéines de (a) ou de (b) en vue de les présenter sur un réseau de bicouches lipidiques.

2. Méthode selon la revendication 1, dans laquelle ladite séquence d'ancrage en 3' est une séquence d'ancrage par le glyocsylphosphatidylinositol (GPI).

3. Méthode selon la revendication 2, dans laquelle ladite séquence d'ancrage par le GPI comprend les 32 acides aminés terminaux de la séquence d'ancrage par le GPI.

4. Méthode selon l'une quelconque des revendications précédentes, dans laquelle les cellules de mammifères sont choisies dans le groupe composé de cellules CHO et HEK-293.

5. Méthode selon l'une quelconque des revendications précédentes, dans laquelle ladite séquence signal est sélectionnée à partir d'une protéine choisie dans le groupe composé du facteur de croissance épidermique, de l'insuline, du facteur de croissance neural, du facteur de croissance dérivé des plaquettes, du glucagon, de l'ICAM-1, du B7-1, du TrkA, du récepteur de facteur dérivé des plaquettes et du CD58.

6. Méthode selon l'une quelconque des revendications précédentes, dans laquelle l'étiquette épitopique de purification est une étiquette épitopique composée d'hexa-histidine.

7. Méthode selon l'une quelconque des revendications qui précèdent, dans laquelle ladite séquence de myristoïlation est une séquence de codage de myristoïlation contenant le gène c-Src.

8. Vecteur d'expression opérable pour la génération d'une protéine à domaine extracellulaire attaché pour présentation sur un réseau de bicouches lipidiques comprenant :
une séquence signal en 5' ;
une étiquette épitopique de purification ;
une séquence codant pour le domaine extracellulaire d'une protéine membranaire ; et
une séquence d'ancrage en 3' pour attacher ou associer le domaine extracellulaire de la protéine membranaire à une membrane à bicouche lipidique située dans un réseau de bicouches lipidiques.

9. Vecteur selon la revendication 8, dans lequel ladite séquence d'ancrage est une séquence d'ancrage par le GPI.

10. Vecteur selon la revendication 8 ou 9, dans lequel ladite étiquette épitopique de purification est une étiquette épitopique composée d'hexa-histidine.

11. Vecteur d'expression pour la génération d'une protéine de domaine intracellulaire attaché pour présentation sur un réseau de bicouches lipidiques consistant en :
une séquence signal en 5' pour la myristoïlation ;
une séquence codant pour le domaine intracellulaire d'une protéine membranaire ; et
une étiquette épitopique de purification en 3'
dans lequel la séquence signal en 5' de myristoïlation permet la présentation du domaine intracellulaire de la protéine membranaire sur un réseau de bicouches lipidiques.

12. Vecteur selon la revendication 11, dans lequel ladie étiquette épitopique de purification est une étiquette épitopique composée d'hexa-histidine.

13. Méthode selon l'une quelconque des revendications 1 à 7 prévoyant en outre de purifier ladite protéine attachée par ancrage.
